# EUROPEAN PATENT SPECIFICATION

(11) **EP 1 360 935 B1**
(45) Date of publication and mention of the grant of the patent: **06.12.2006**
(21) Application number: 03252884.6
(22) Date of filing: 08.05.2003
(51) Int. Cl.: A61B 5/15

(54) **Strip containing a series of fluid sampling and testing devices and method of making, packaging and using it**
Streifen zur Verpackung einer Mehrzahle von Geräten zur Flüssigkeitsentnahme und Testung sowie Verfahren zur Herstellung und Verwendung des Streifens
Band contenant des appareils pour la collecte et l'analyse d'échantillons de fluid et méthodes pour la produire, l'utiliser et l'emballer

(30) Priority: 09.05.2002 US 142409
(43) Date of publication of application: 12.11.2003
(73) Proprietor: LifeScan, Inc., Milpitas, CA 95035-6312 (US)
(72) Inventor: Olson, Lorin, Scotts Valley, California 95066 (US); McAllister, Devin, Shrewsbury Massachusetts 01545 (US)
(74) Representative: Mercer, Christopher Paul

(56) References cited:
- WO-A-01/72220
- DE-A- 19 819 407
- DE-B- 2 803 345
- US-A- 4 787 398
- US-A- 5 776 157
- US-A- 6 093 156

## Description

### FIELD OF THE INVENTION

This invention relates to the sampling and testing of physiological fluid. More particularly, the invention relates to continuous strips of physiological fluid sampling and testing devices, the manufacturing thereof and the use thereof within a meter configured for individually dispensing such sampling and testing devices.

### BACKGROUND OF THE INVENTION

Analyte concentration determination in physiological samples is of ever increasing importance to today's society. Such assays find use in a variety of application settings, including clinical laboratory testing, home testing, *etc.,* where the results of such testing play a prominent role in the diagnosis and management of a variety of disease conditions. Analytes of interest include glucose for diabetes management, cholesterol for monitoring cardiovascular conditions, drugs for monitoring levels of therapeutic agents, and identifying illegal levels of drugs, and the like. In response to this growing importance of analyte concentration determination, a variety of analyte concentration determination protocols and devices for both clinical and home testing have been developed.

In determining the concentration of an analyte in a physiological sample, a physiological sample must first be obtained. Obtaining and testing the sample often involves cumbersome and complicated procedures. Unfortunately, successful manipulation and handling of test elements, such as test strips, lancing members, meters and the like is to a great extent dependent on the visual acuity and manual dexterity of the user, which in the case of people with diabetes is subject to deterioration over the course of the disease state. In extreme cases people that have significant loss of sight and sensation, testing procedures can become significantly difficult and requires additional assistance from ancillary devices or personnel.

A typical procedure for making a glucose measurement with the use of a test strip involves the following actions or steps (but not necessarily in the order given): (1) removing supplies from a carrying case, (2) removing a lancing device loading cap or door, (3) removing and disposing of a used lancet from the lancing device, (4) inserting the lancet in the lancing device, (5) twisting off a protective cap from the lancet, (6) replacing the lancing device cap, (7) cocking the lancing device, (8) opening a test strip vial/ container, (9) removing a strip from the container and inserting or interfacing it with a meter, (10) holding a lancing device to the skin, (11) firing the lancing device, (12) removing the lancing device from the skin, (13) extracting a sample, (14) applying sample to the test strip and obtaining results of the measurement; (15) disposing of the test strip, (16) cleaning the test site, and (17) returning supplies to the carrying case. Of course, certain glucose measurement systems and protocols may involve fewer or more steps.

One manner of reducing the number of actions is by the use of integrated devices which combine multiple functions in order to minimize the handling of sensor and/or lancing components which may lead to contamination of the components and /or injury to the user. In this regard, certain test strip dispensers are configured to both store and advance successive test strips upon actuation. Examples of such devices are presented in U.S. Patent Nos. 5,510,266; 5,660,791; 5,575,403; 5,736,103; 5,757,666; 5,797,693; 5,856,195 and PCT Publication WO 99/44508. Some of these test strip dispensers devices also include meter functionality for testing physiologic fluid.

Another class of devices designed to decrease the number of steps required in test strip use includes automatic or semi-automatic lancing devices. U.S. Patent No. 6,228,100 discloses a structure configured for sequential firing of a number of lancets, one at a time, in order to eliminate the requirement that a user remove and replace each lancet individually before and after use. However, this device does not include any sensor components or functions. While other systems, such as those described in U.S. Patent Nos. 6,352,514; 6,332,871; 6,183,489; 6,099,484; 6,056,701 and 5,820,570, integrate the test strip and lancing functions within a single device, the test strip and lancet are separate components which must be loaded into the meter or dispenser individually.

The device disclosed in U.S. Patent No. 5,971,941 attempts to combine the functionality of each of the preceding classes of test strip devices. In effort to provide an "integrated" system for sampling blood and analysis thereof, it includes a magazine of test strips, test strip advancement and dispensing features, a meter with a display and an automated lancing mechanism all housed within a single box. While presenting some measure of improvement in user convenience, the test strip and lancing features are independent from each other causing the user to take two steps in lancing and transferring sample to a test strip.

WO 02/49507 discloses a meter including a microneedle which extracts fluid from a subject and is selectively switched between multiple sensor elements. While offering a slight improvement over prior devices, such configurations typically require complex componentry which can be difficult to operate and are costly to produce. Further, the more componentry and moving parts within a device, the more questionable the reliability of the device.

Technological advancements have been made in test strip fabrication where both sensor and lancing functions and structures are provided on a single test strip. Certain web-based methods have been used to make such fully integrated test strips which are singulated after fabrication prior to being collectively packaged in a cartridge, magazine, cassette or the like. While such test strips packaging reduces, to some degree, the necessary handling by a user, this modality still requires additional manufacturing and packaging steps.

While many of the above-described devices and other similar devices involve changing-out spent test strips and/or lancet members one-at-a-time, such as those described in U.S. Patent Nos. 6,027,459; 6,063,039; 6,071,251 and 6,283,926 as well as for certain embodiments disclosed in PCT Publication WO 01/64105, certain other devices provide means for ejecting a used step strip or dispensing it into a receptacle within the device. Still other devices provide for multiple lancet/sensor pairs that only need to be changed out after all of the test strip and/or lancet members within a cartridge or cassette are used.

As such, there is continued interest in the development of new devices and methods for use in test strip dispensing. Of particular interest would be the development of such devices and methods which are easy and inexpensive to manufacture, have minimal components, are portable and easy to use, particularly for visually and dextrally impaired individuals, and involve no direct contact and minimal handling by the user, thereby minimizing the risk of injury to the user and damage and/or contamination to the test strip devices contained therein.

Of course, such advantages may be present in the devices and systems of the present invention in various degrees. It is intended that, in one way or another, the invention is of assistance in reducing barriers to patient selfmonitoring and therefore result in improved outcomes in the management of disease, such as diabetes.
Document DE 2803345 discloses a device as defined in the preamble of claim 1.

### SUMMARY OF THE INVENTION

The present invention provides continuous strips of testers in an individually-sealed packaging which maintains the testers in a sterile condition until used. The subject testers include a test strip sensor integrated with a microneedle for accessing and collecting a sample of physiological fluid and for measuring a chemical characteristic, such as target analyte concentration, of the sampled fluid. The present invention further provides cassettes of the subject strips for removable engagement with a meter for individually dispensing the testers and for facilitating the analysis of the sampled fluid. Also provided by the present invention are systems which include the subject strips and cassettes and such a meter.
The present invention is as defined in the appended claims.

An aspect of the present invention is to provide devices and methods for facilitating a user in the self-testing of analyte concentrations, such blood glucose levels. More particularly, such devices facilitate the easy, convenient and safe dispensing of testers for the measuring of analyte concentrations and the subsequent storage of such testers after they have been used.

These and other objects, advantages, and features of the invention will become apparent to those persons skilled in the art upon reading the details of the methods and systems of the present invention which are more fully described below.

### BRIEF DESCRIPTION OF THE FIGURES

To facilitate understanding, the same reference numerals have been used (where practical) to designate similar elements that are common to the Figures. Some such numbering has, however, been omitted for the sake of drawing clarity.

Fig. 1A is a planar view of one embodiment of according to the present invention of a strip of singulated testers. Fig. 1B is a side view of the strip of Fig. 1A taken along the line B-B.

Fig. 2A is a planar view of another embodiment according to the present invention of a strip of interconnected testers. Fig. 2B is a side view of the strip of Fig. 2A taken along the line B-B.

FigS. 3A-3K illustrate various steps of a continuous web-based method of the present invention for fabricating the strip of testers of Figs. 1A and 1B.

Fig. 4A is an isometric view of a cassette of the present invention for storing a strip of testers of the present invention.

Fig. 4B is an isometric view of the front side of a meter of the present invention for use with the cassette of Fig. 4A.

Fig. 4C is an isometric view of the cassette of Fig. 4A in the process of engagement with the meter of Fig. 4B.

Fig. 4D is an isometric view of the cassette of Fig. 4A fully engaged within the meter of Fig. 4B.

Fig. 5 is a cross-sectional planar view of the cassette of Fig. 4A.

Fig. 6 illustrates a strip of testers operatively engaged with a portion of the internal componentry of a cassette of the present invention.

Figs. 7A, 7B and 7C are various views of a distal portion of the meter housing which interfaces with the testers as they are dispensed.

Fig. 8 provides a view of a cutaway portion of the cassette of Fig. 5 engaged within the meter of Figs. 4B-D.

### DETAILED DESCRIPTION OF THE PREFERRED EMBODIMENTS

Before the present invention is described in such detail, it is to be understood that this invention is not limited to particular variations set forth herein as various changes or modifications may be made to the invention described and equivalents may be substituted without departing from the true spirit and scope of the invention. In addition, many modifications may be made to adapt a particular situation, material, composition of matter, process, process act(s) or step(s) to the objective(s), spirit or scope of the present invention. All such modifications are intended to be within the scope of the claims made herein.

Methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

All existing subject matter mentioned herein (*e.g.*, publications, patents, patent applications and hardware) is incorporated by reference herein in its entirety except insofar as the subject matter may conflict with that of the present invention (in which case what is present herein shall prevail). The referenced items are provided solely for their disclosure prior to the filing date of the present application. Nothing herein is to be construed as an admission that the present invention is not entitled to antedate such material by virtue of prior invention.

Reference to a singular item, includes the possibility that there are plural of the same items present. More specifically, as used herein and in the appended claims, the singular forms "a," "and," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Last, it is to be appreciated that unless defined otherwise, all technical and scientific terms used herein have the same meaning as commonly understood by one of ordinary skill in the art to which this invention belongs.

In further describing the subject invention, the subject packaged testers and methods of fabricating them are described first. Next, a description of the subject cassettes and methods of using them with a physiological fluid meter is provided. Finally, a review of the kits of the present invention which include the packaged testers and/or the cassettes is provided.

In the following description, the present invention will be described in the context of analyte concentration measurement applications, and particularly in the context of glucose concentration in blood or interstitial fluid; however, such is not intended to be limiting and those skilled in the art will appreciate that the subject devices, systems and methods are useful in the measurement of other physical and chemical characteristics, *e.g.,* blood coagulation time, blood cholesterol level, the existence of legal or illegal drugs, etc. of other biological substances, *e.g.,* urine, saliva, etc.

### Devices of the Present Invention

As mentioned above, the strips of the present invention provide a plurality of testers. Prior to a detailed discussion of the strip devices of the present invention, a general description of testers suitable for use with the present invention is provided.

### Testers

The testers may have a configuration similar to that of test strips used for analyte concentration determination, such as in the field of blood glucose monitoring. As used with the present invention, each tester includes a biosensor or sensor portion and an integrated microneedle or lancet. Electrochemical type sensors are most suitable for use with the present invention, however, other suitable types of sensor configurations may be used. Each tester includes two spaced-apart electrodes, a bottom electrode and a top electrode wherein at least one electrode is formed on an inert substrate material. Between the electrodes is an insulating space layer. The spacer layer has a cut-out portion which defines the reaction zone of the electrochemical biosensor containing a redox reagent system. The microneedle extends from a front end of and is planar with one of the electrodes. A channel is formed in the microneedle and a portion of the electrode from which extends, wherein fluid accessed within the skin, *e.g*., blood, interstitial fluid, other body fluids, is transported by the channel into the reaction zone of the electrochemical cell defined by the electrodes. Examples of such testers or test strip devices suitable for use with the present invention include those described in PCT Patent Application No. WO 96/72742/001 claiming priority to Great Britain Patent Application No. GB 0330929.4, EP-A-1 281 352, EP-A-1 284,121 and copending European application No. , claiming priority from USSN 10/143,399 filed 9th May 2002 [Attorney ref: P033762EP].

### Strips of Testers

The strips of the present invention provide a plurality of the above described testers in a serial arrangement where all of the testers are oriented in the same direction. In addition to such serial arrangement and unidirectional orientation, the subject strips are "continuous" over a suitable and practical length for achieving the objectives of the present invention. The testers and the associated strip are preferably fabricated by means of web-based processes wherein the resulting product is a collection of laminated layers of various materials. The testers are contiguous with the strip in that they are either affixed to a strip structure or are integrally fabricated with the strip structure, *e.g.*, a layer of the testers also serves as the strip structure.

The strip structure includes a flexible frame which serves to interconnect the testers in a serial arrangement. The flexibility of the frame allows it to be folded or rolled upon itself for compact storage. The frame includes at least one planar surface, edge or ledge that extends the length of the strip and is integral with or affixed to each tester. Most commonly, the frame includes two planar edges which straddle the serially aligned testers and further includes a planar surface which bridges the two edges. As such, in a flat or unfolded or rolled condition, the testers substantially lie in the same plane as the frame. The testers are flexibly attached to or integral with the frame structure such that the testers are movable, flexible or bendable from the plane defined by the frame structure.

The strip structure may further include a flexible packaging material which hermetically seals the strip to maintain the testers in a sterile condition prior to use. Such packaging is also in the form of a strip or strips having length and width dimensions commensurate with that of the frame. In certain embodiments, two strips of packaging material are provided, which are heat sealed on either side of the strip structure and the associated test strips. Additionally, the strip structure may further provide recesses or pockets within which the testers individually reside. In certain embodiments, the recess or pocket may be formed by the packaging material itself.

The strip structures, with or without the packaging, may be provided within a cassette which is configured to be engagable or loadable within a meter for analyzing the physiological fluid samples collected by the testers wherein the meter is configured to individually dispense the testers for use. Alternatively, the strip may itself be configured to be engagable or loadable within the meter.

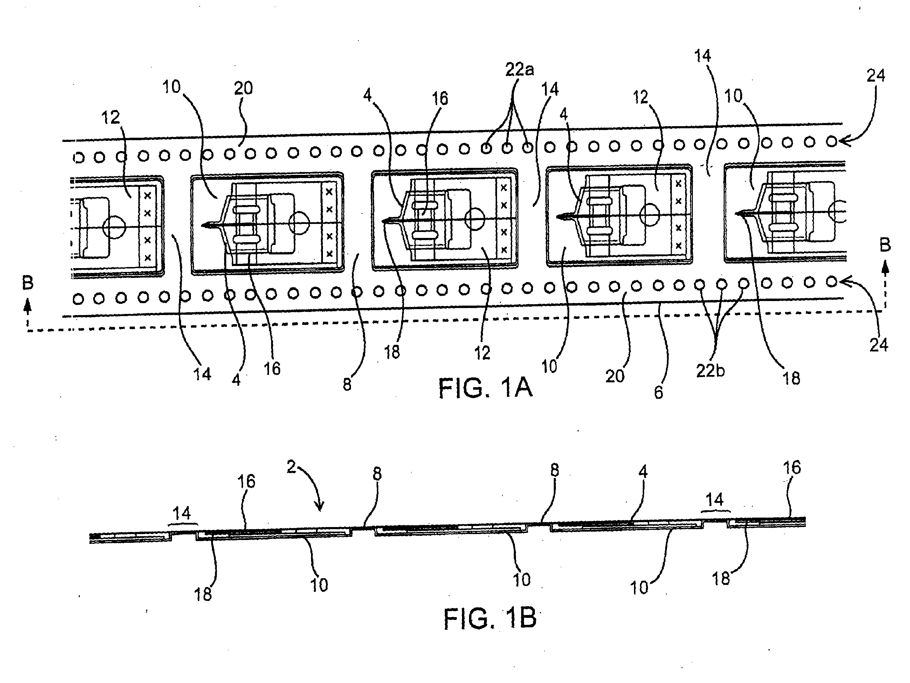

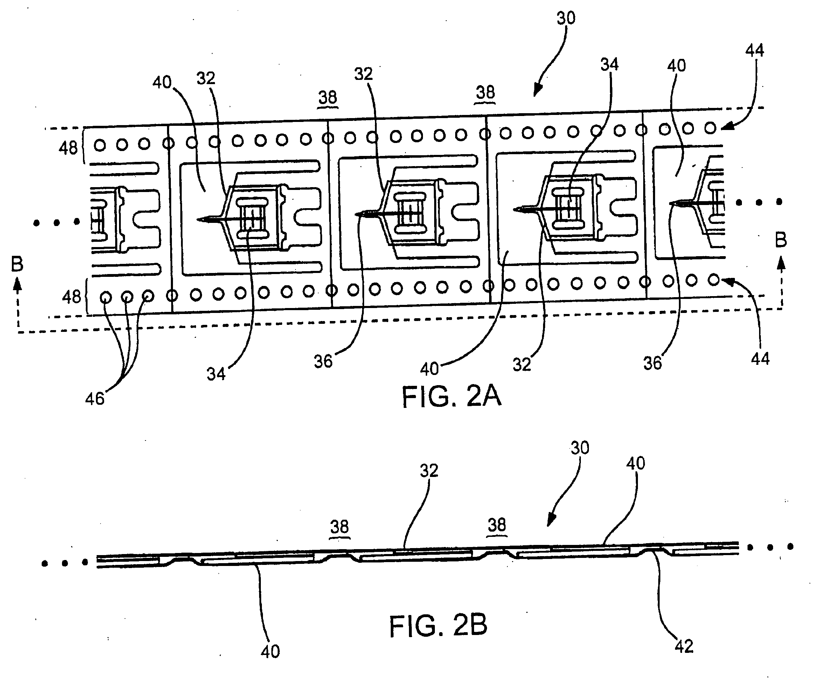

Referring now to the Figures and to Figs. 1A, 1B, 2A and 2B in particular, there are shown two embodiments of the strips of testers of the present invention. While the construct of the two embodiments of strips are similar in that both provide a continuous serial arrangement of testers which are in a contiguous structure, there are differences. The embodiment of Figs. 1A and 1B provide for testers which are singulated from each other during the manufacturing process and then affixed to a separately fabricated strip structure. Conversely, the strip embodiment of Figs. 2A and 2B provides for testers which are interconnected to each other by at least one webbing of material used to fabricate the testers, which webbing material serves to also form at least a portion of the strip structure or frame.

Strip 2 of Figs. 1A and 1B provides a plurality of testers 4, as described above, which are singulated from each other and are individually positioned within a frame structure 6. As mentioned, each tester 4 has a sensor portion 16 and a microneedle or lancet 18 which extends from sensor portion 16. Strip 2 further includes a double-sided packaging, having a bottom, tray or recessed portion 8 and a top or cover portion which is not shown for purposes of illustration. Bottom portion 8 has a tray configuration within which is formed a plurality of recesses 10, otherwise referred to as blisters, pockets or cavities, having length, width and depth dimensions for accommodating or containing testers 4. The top portion of the packaging includes a thin flexible sheet of material and, when operatively affixed to tray 8, individually seals each of recesses 10. As such, the packaging preserves the sterility of each recess 10, provides a moisture barrier for each recess, and prevents cross-contamination between recesses. Suitable packaging materials are discussed in greater detail below.

Each recess 10 has a length in the range from about 1 to 50 mm and more typically from about 10 to 20 mm; a width in the range from about 1 to 30 mm and more typically from about 5 to 15 mm; and a thickness or depth in the range from about 100 to 3,000 µm and more typically from about 500 to 1,000 µm. While recesses 10 are illustrated and described as being rectangular, any suitable shape, *e.g.*, square, oval, elliptical, circular, etc., and configuration may be employed. Preferably, there is a one-to-one correspondence between the number of testers 4 and the number of recesses 10. Strip 2 may provide any number of testers 4 (and corresponding number of recesses 10) suitable for the application at hand. Typically, for monitoring glucose levels in a diabetic, for example, strip 2 may provide about 10 to 50 testers/recesses.

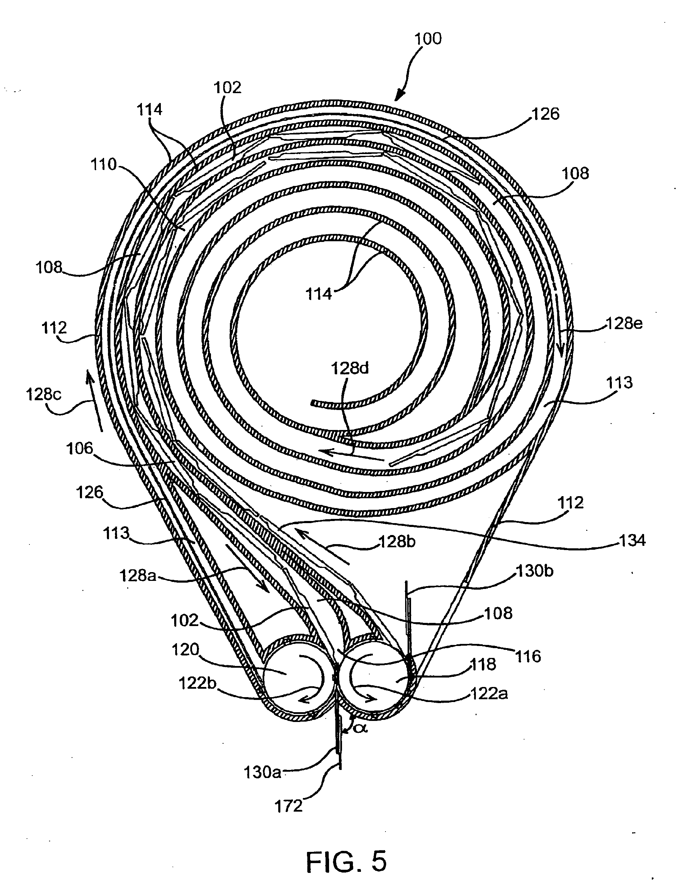

The proximal end 12 of each tester 4 is affixed to the recess 10 within which a tester 4 is contained. As such, when the recess is not covered or sealed, each tester 4 is flexible, bendable, deflectable or movable through the opening and to the outside of recess 10 about an axis defined by proximal end 12. Such range of motion is defined by an angle α (see Fig. 5) having a minimum range of motion no less than about 45°, and typically no less than about 60°. Alternatively or additionally, the bottom portion of the packaging material may be flexible or pre-scored to promote flexibility between recesses such that frame 6 and testers 4 are bendable or deflectable relative to each other.

Frame 6 includes a planar surface defined around the opening of each recess 10. This planar surface defines a narrow margin 14 which extends between adjacent recesses 10 and defines a side edge or ledge 20 which extends the length of strip 2. Each bridge 14 is flexible about its longitudinal axis (*i.e.*, along the width of strip 2) wherein adjacent testers are bendable about such an axis. Recesses 10 are preferably evenly spaced from each other along the length of strip 2. Along each side or edge 20 of strip 2 and extending the length of strip 2 is a row 24 of evenly spaced, serially aligned sprocket holes 22 for receiving drive sprockets of a drive wheel, discussed in greater detail below.

Figs. 2A and 2B illustrate another strip 30 of the present invention providing a frame 42 and plurality of testers 32 flexibly attached to frame 42. Each tester 32 has a sensor portion 34 and a microneedle or lancet 36, as described above. Testers 32 of strip 30 are interconnected to each other by at least one continuous web or layer of material, *e.g.*, an inert substrate material, a spacing layer, etc, from which testers 32 are made, or the interconnection may be provided by an additional layer which is not used to form testers 32. Such continuous web or layer of material defines frame 42 and includes segments 38 extending between or bridging adjacent testers 32. As described above with respect to frame 6, frame 42 has side rows 44 of holes 46. A spacing 40 is defined between each tester 32 and frame 42.

### Device Fabrication Methods

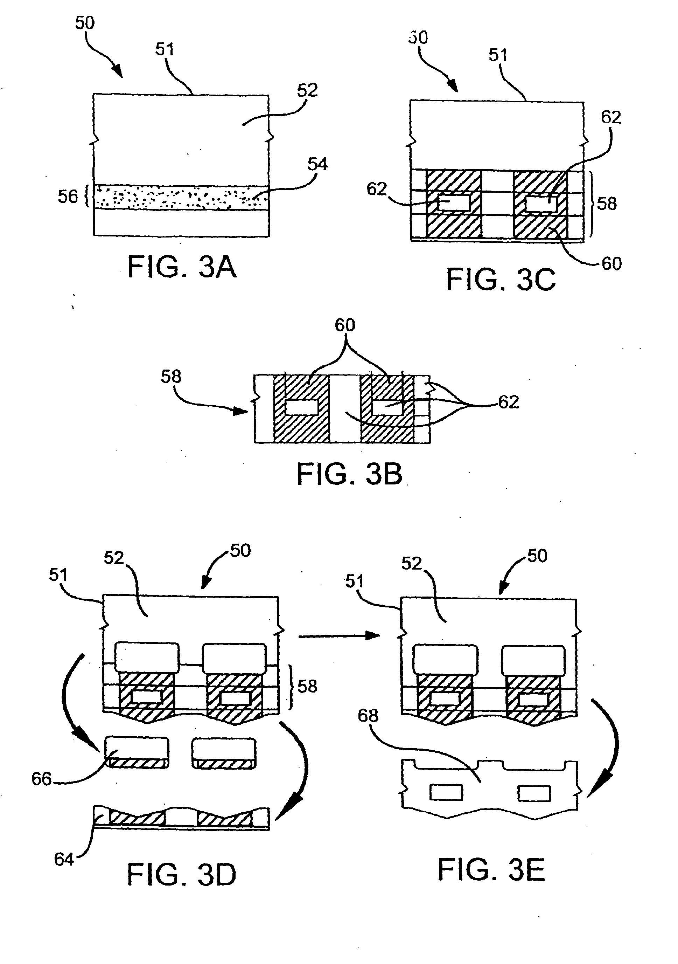

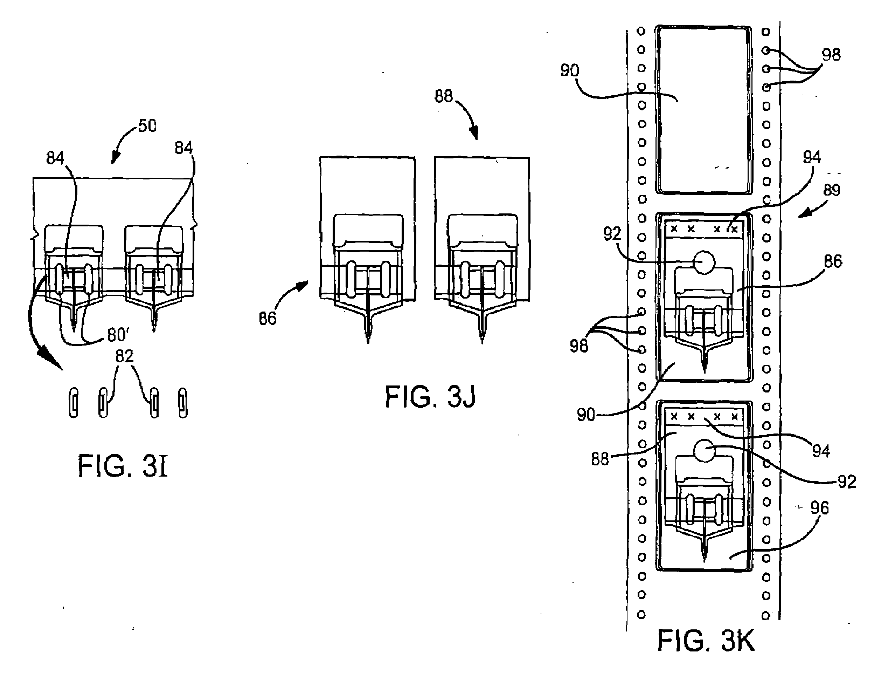

Referring now to Figs. 3A-3M, there is shown certain steps of a web-based fabrication process for fabricating the strips of the present invention and, in particular, strip 2 and singulated testers 4 of Figs. 1A and 1B. Those skilled in the art will appreciate the possible variations in these steps, or the addition or subtraction of one or more steps, in order to fabricate strip 30 and the interconnected testers 32 of Figs. 2A and 2B. For example, while the singulated testers 4 are fabricated in a side-to-side arrangement along the length of webbing, the interconnected testers 32 are fabricated in an end-to-end arrangement along the length of webbing.

For purposes of describing a web-based fabrication process of the present invention, only two testers are illustrated in Figs. 3A-3K; however, it is appreciated that any number of testers may be concurrently fabricated along the same length of webbing. Also, for purposes of describing the present invention, the same reference number (50) shall be throughout Figs. 3A-3K to identify the cumulative webbing structure which results from each successive step of the fabrication process.

The fabrication process is initiated by providing a continuous webbing of substrate material 51 upon which a first conductive layer 52 is provided to form a first electrode, collectively forming a cumulative webbing structure 50. The substrate webbing 51 preferably consists of a polymer film, a foil material and/or inorganic materials such as silicon, glass, ceramic and the like. A suitable polymer, for example, is polyester, *e.g.*, polyethylene terephthalate (PET). Conductive layer 52 may be provided by any one of a variety of techniques including, but not limited to, vacuum sputtering, evaporation techniques, *e.g.*, electron beam evaporation, filament evaporation, etc.), electroplating, electroless plating, screen printing and the like. Suitable conductive materials include, but are not limited to, palladium, gold, platinum, silver, iridium, stainless steel and the like, or a metal oxide, such as carbon, *e.g.*, conductive carbon ink, or doped tin oxide; however, for purposes of describing the present invention, palladium is used as conductive layer 52. Thereafter, as shown in Fig. 3A, a redox reagent system 54 is deposited within a reaction zone 56 of palladium layer 52 along the length of webbing structure 50. Such deposition may be accomplished with slot coating, needle coating, screen printing or ink jet printing techniques, which are well known in the art.

In an ancillary web-based process, a spacer layer webbing 58, as shown in Fig. 3B, is provided having a spacer layer of which both surfaces have an adhesive layer applied thereto. These three layers are collectively sandwiched between bottom and top protective release liners. The resulting 5-layer spacer webbing 58 is then selectively "kiss" cut so as only to cut through portions of the top release liner, the adhesive layers and the spacer layer, but not through the bottom release liner. The portions of the top release liner and the adhesive layers which are cut free are removed from spacer webbing 58, as illustrated in Fig. 3B, what remains are the exposed portions 60 (the hatched areas) of the adhesive layers and the bottom release liner. Finally, as illustrated in Fig. 3C, the top side or surface of the resulting spacer layer webbing 58 is then applied over the reagent zone 56 of webbing structure 50, adhering thereto by means of the exposed areas 60 of the bottom adhesive.

As illustrated in Fig. 3D, cumulative webbing structure 50 is then selectively die cut completely through its various web layers, *i.e.*, bottom release liner (now the top layer of cumulative webbing structure 50), adhesive layer and palladium layer 52. Specifically, the front or forward portion 64 of webbing structure 50 is cutaway to prepare webbing structure 50 for receiving the microneedle webbing. Additionally, a scrap piece 66 is cutaway from each tester The cut out portions 64 and 66 are then removed from webbing structure 50 and scrapped. Next, the remaining release liner 68 is peeled away from webbing structure 50 and discarded, as shown in Fig. 3E.

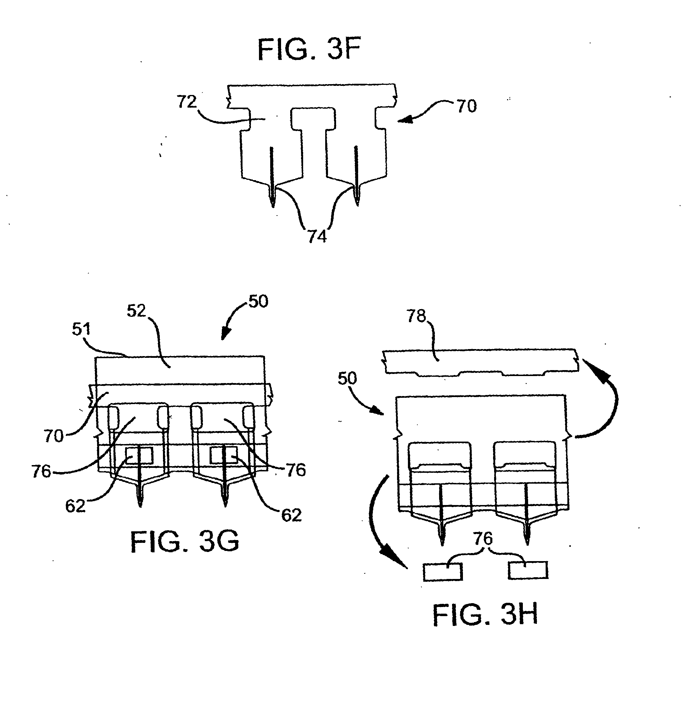

In another ancillary web-based process, as illustrated in Fig. 3F, a webbing of conductive material 70 is provided and die cut to form the second electrode layer 72 and microneedles 74. Conductive material webbing 70 may include any of the conductive materials used above for fabricating the first conductive layer 52; however, for purposes of describing the invention, gold-plated stainless steel is used. As illustrated in Fig. 3G, conductive material webbing 70 is then placed on top of cumulative webbing structure 50, which structure now includes a substrate material 51 on the bottom thereof, and palladium layer 52 having a reagent thereon, the previously exposed, remaining adhesive layer 60, spacer creating layer 62 and gold-plated stainless steel layer 72, successively stacked thereon.

Next, as shown in Figs. 3H and 3I, several die cuts are made to cumulative webbing structure 50. First, bridges 76 formed by the gold-plated stainless steel layer 72 are die cut and scrapped. Cutting bridges 76 also frees piece 78 of layer 72 which is also scraped. Next, a die cut is made to remove two scrap pieces 82 on opposing sides of reaction area 84 to provide a venting port within the electrochemcial cell and to define an exact volume for sample collection and a precise electrode surface area. Finally, to complete the tester fabrication process, cumulative webbing structure 50 is cut across its width between adjacent testers, resulting in a plurality of singulated testers 86 and 88.

After the plurality of testers 86 and 88 is fabricated, they are picked and placed by robotic means within the bottom portion of a packaging 89 in a serial arrangement, resulting in the strip configuration described above with respect to Figs. 1A-2B. The bottom or tray portion of the packaging strips of the present invention is preferably made of flexible material but may be rigid instead. Suitable materials include plastics, *e.g.*, high-density polyethylene, having an external surface formed of aluminum foil-plastic laminate integrally bonded to the plastic tray. The tray structure, including recess cavities 90 and the planar surfaces around the recess openings, may be formed by compressing the material between matched dies. For strip embodiments having interconnected testers, wherein the resulting tester webbing provides the interconnected testers in an end-to-end arrangement, the entire strip is placed on top of the bottom portion of the packaging, wherein each tester is centrally aligned with a recess.

Either before or after placement or alignment of the testers within the bottom portion of the packaging, a desiccant material 92 is disposed on, and preferably adhered to, each tester at a location that will not interfere with the sensor and microneedle functions. The desiccant material 92 helps to maintain each recess at an appropriate humidity level after packaging so that the sensor chemistry is not adversely affected prior to use. Because the recesses, as described below, are hermetically isolated from each other, the opening of one recess will not affect the hermetically sealed state of the other recesses. The desiccant material may have any suitable form, including but not limited to a disk or bead configuration.

Next, or prior to inclusion of the desiccant material within the recesses, the proximal end 94 of each tester is attached to and subsequently retained within the interior of the recess in which it is positioned. The testers are attached by means of sonic welding, an adhesive material, or the like, as it is intended that each tester be permanently retained within or associated with the recesses to which it is attached for the operative life of the tester.

A top portion (not shown) of packaging 88 is provided which has length and width dimensions commensurate or similar with that of the bottom portion of packaging 89 and may be made of materials similar to that of the bottom portion of the packaging, such as a plastic laminate having aluminum liner. The top portion is then aligned on the top of bottom or recess portion of packaging 88 and heat sealed therewith along the perimeters of each recess, thereby forming a laminated packaged strip having a plurality of sealed recesses encased in a moisture and sterility barrier. Finally, sprocket holes 98 are die cut on both edges of the packaged strip. The resulting packaged strip is now ready for operative loading into a cassette or cartridge of the present invention, which will now be described in detail.

### Systems of the Present Invention

As mentioned above, the subject strips may be configured to be loaded within a cassette which is configured to be loaded within a meter for analyzing physiological fluids. Generally, the meter is configured to receive the cassette and provided with a mechanism which engages with and indexes the strip. The strip is provided with sprocket holes to engage with a pair of indexing rollers within the meter which are configured to index and dispense only a single tester at a time upon activation of the meter by the user. The indexing and dispensing of the flexibly attached testers involves peeling back the packaging material, if such is provided, and moving the tester in a manner and into a position for piercing the skin surface.

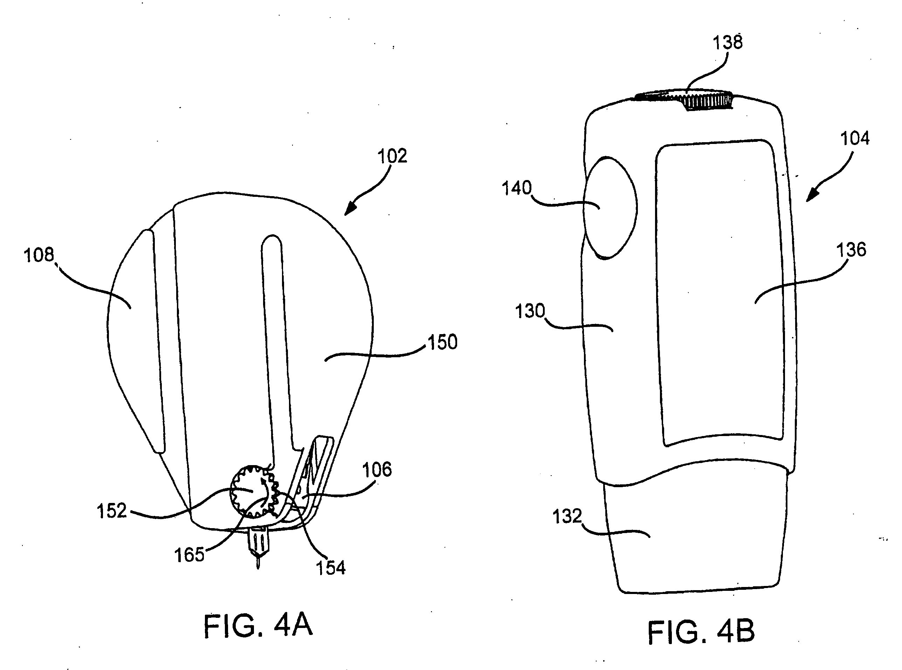

Referring now to Figs. 4A-4D, a system of the present invention is illustrated. System 100 includes a cassette or cartridge 102 and a meter 104. Cassette 102 contains a strip 106 of testers (see Fig. 4B), as described above with respect to Figs. 1A, 1B, 2A, 2B and 3K, operatively loaded therein. Cassette 102 and meter 104 are configured to operatively engage with each other wherein meter 104 functions to individually dispense testers of strip 106 from cassette 102 and to measure one or more characteristics, *e.g*., one or more analyte concentrations, of the body fluid sampled by a dispensed tester. Each of the cassette 102 and meter 104, their operative engagement and collective operation and function are now described in greater detail.

### Cassettes

Cassette 102 has a modular configuration and may be replaced and disposed of upon use of all testers contained within cassette 102. Additionally, cassette 102 is configured in any convenient manner to accommodate a strip 106 of the present invention containing a plurality of testers. The number of testers within a single strip or loop is sufficiently large, *e.g.*, between about 10 and 100, and more typically between about 10 and 50, so as to minimize the frequency with which the user must replace the cassette, and preferably, not so numerous such that the testers' effectiveness expires prior to being used.

Cassette 102 may contain a barcode or some other means (such as a chip) for transferring information to meter 104 upon operatively engaging loading the cassette with the meter. As such, meter 104 would automatically read this information when cassette 102 via a detection system. Any conventional detector may be employed. Information that may be useful includes: a calibration factor or code for calibrating the meter according to the type of testers employed within the cassette, the number of testers used and/or remaining within the cassette, the number of days since the cassette was installed and/or days until tester expiration. The latter information is important as the testers (within a packaged strip) have a limited lifetime.

Cassette housing 102 is preferably light-weight, ergonomically designed and small enough to be handled by the user with one hand, but which is spacious enough internally to accommodate strip 106 as well as testers which have been used (in operation with the meter) and the associated packaging material from such used testers. One useful cassette configuration is illustrated in Fig. 5. This configuration provides an internal construct having a set of curvilinear tracks or grooves 108, 110 and 113 defined and separated by walls 114, which run substantially parallel to each other in a wound or spooled configuration. The tracks have width dimensions to accommodate the thickness of a strip recess and a wall height to accommodate the strips' width. Walls 114 are sandwiched between two sides of housing 112. Such a design is preferably made of a plastic material, such as ABS or any other suitable thermoplastic, and fabricated by injection molding techniques.

The tracks originate from a centralized area of cassette 102 and terminate at a peripheral portion of cassette 102 at a pair of drive rollers 118 and 120. A first or central track 108 runs between second or inside track 110 and third or outside track 113. Central track 108 is used to accommodate a packaged strip 106, which is preferably pre-loaded within cassette 102 during the manufacturing assembly process. Central track 108 terminates at a spacing or throat 116 between internal cylindrically-shaped drive rollers 118 and 120. The distance between drive rollers 118 and 120 which define margin or dispensing space 116 is sufficient to snugly accommodate the thickness of a packaged recess of strip 106 there through without compressing the recess, so as not to cause damage to the tester therein. As shown in Fig. 4A, drive roller 118 is axially engaged through housing 108 of cassette 102 with a drive gear 152 positioned on the backside 150 of cassette 102. Drive gear 152 has teeth 154 for engagement with internal componentry of meter 104, described in greater detail below, for rotating drive roller 118. In turn, drive roller 118 rotates drive roller 120 by sprocket pins in the direction of arrow 122b by means of sprocket pins (not shown) which extend radially from roller 118 into corresponding pin recess (not shown) within roller 120. Alternately, both rollers may provide pins and recesses which correspond to the recesses and pin, respectively.

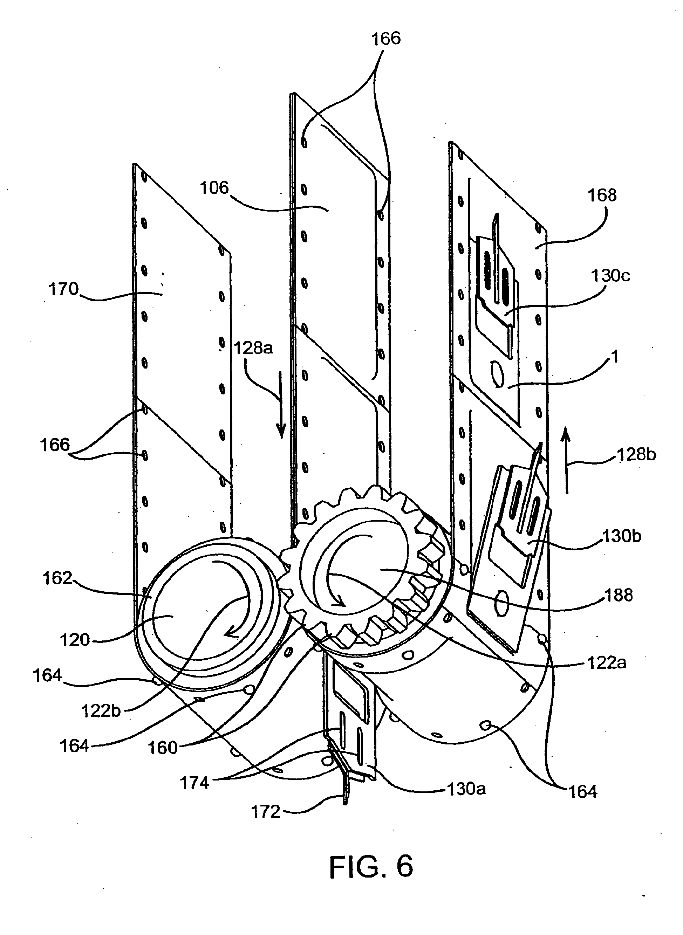

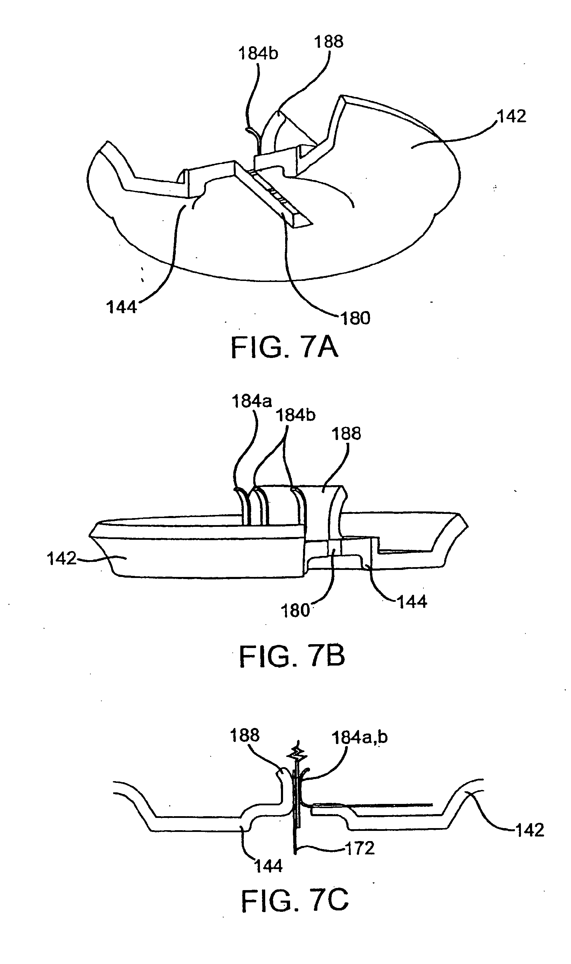

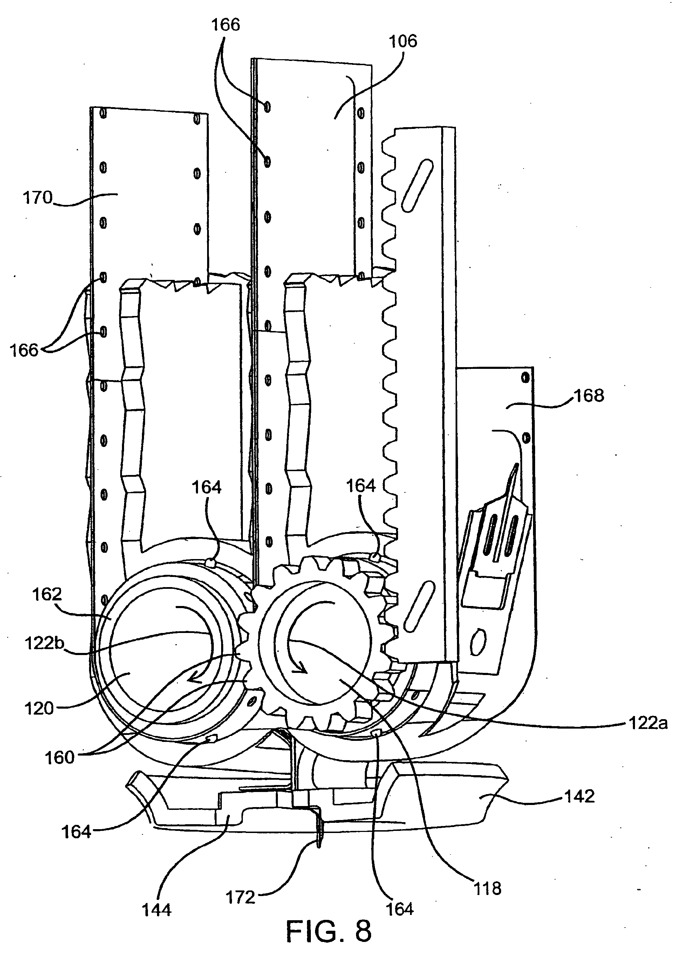

As shown in Fig. 6, each of rollers 118 and 120 have radially-extending sprockets 164 which are each sized and spaced apart from each other an equidistance to matingly engage with holes 166 of packaged strip 106. Thus, as rollers 118 and 120 rotate towards each other, sprockets 164 mate with holes 166 and cause packaged strip 106 to be indexed or advanced in the direction of arrow 128a (also shown in Fig. 5). By indexing it is meant that the strip is caused to advance only a fixed distance and expose and dispense only a single tester upon activation of the meter's mechanics to perform a fluid sampling and measurement routine. Such indexing routine and the corresponding meter mechanics will be described in greater detail below. The resulting indexing motion causes the strip packaging to pull apart or separate wherein the bottom or recess portion 168 of the packaging remains engaged with roller 118 and is caused to advance in the direction of arrow 128b and wherein the top or cover portion 126 portion remains engaged with roller 120 and is caused to advance in the direction of arrow 128c. As such, tester 130a becomes exposed as it passes through rollers 118 and 120 and is flexed away from its recess, extends downward in a direction substantially perpendicular to the rotation of rollers 118 and 120 and substantially normal to the skin surface. In this distally extended or dispensed position, tester 130a is in a testing or active position wherein microneedle 172 is caused to penetrate the skin and access the targeted physiological fluid therein and the electrode contacts 174 of tester 130a are positioned to contact corresponding electrical contacts of meter 104, which will be discussed in greater detail below with respect to Figs. 7 and 8.

Repeated indexing of strip 106 results in successive use of the testers therein and advances bottom or recess portion 168 and top or cover portion 170 of the strip packaging into tracks 110 and 113, respectively, in the direction of 128d and 128e, respectively. As each tester is heat sealed to the inside of the recess in which it is packaged, each successive indexing routine advances the most recently used tester along with bottom packaging portion 168, as illustrated by tester 130b of Figs. 5 and 6. As the testers and recesses achieve a substantially parallel indexing path, the testers become nested in their corresponding recess, as illustrated by tester 130c of Figs. 5 and 6.

### Meters

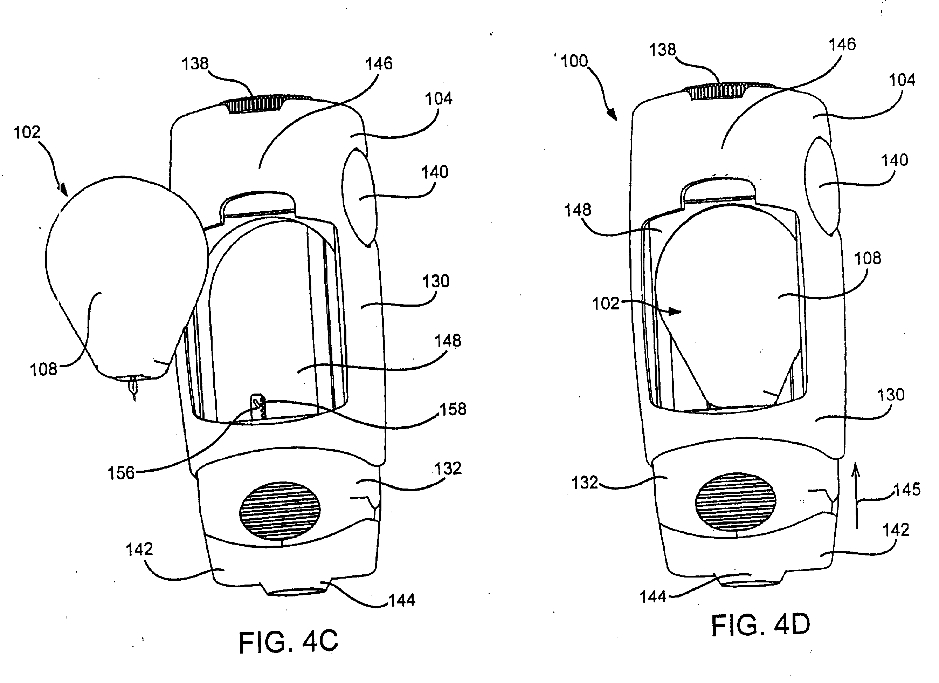

Meter 104 of system 100, its engagement with cassette 102 and its operation are now described with respect to Figs. 4B-4D, 7A-7C and 8. Referring to Figs. 4B-4D, meter 104 includes an upper body portion 130 and a lower body portion 132, the latter of which may be covered by a protective cap (not shown) when not in use, and is exposed upon removal of the cap. Upper body portion 130 provides a display screen 136 and various knobs 138 and buttons 140 for user control and operation of meter 104. Additional buttons may be provided to allow the user to interact with the meter, *e.g.*, scroll through data, set time and date, etc. Display screen 136 may be used to display various user directions, system messages and data, *e.g*., analyte measurement values, the number of unused testers remaining, the number to expiration of the unused testers, *etc.* The backside 146 of upper body portion 130 includes an open receptacle or cavity 148 configured and dimensioned for receiving cassette 102. As shown in Fig. 4C, receptacle 148 includes a geared tooth rack 156 having drive teeth 158 which engage with gear teeth 154 of drive wheel 152.

Lower portion 132 of meter 104 includes a distal housing face 142 having a distally extending "pressure ring" 144 which, when applied to the target skin surface, depresses tissue around a periphery of the intended access site within the skin. All or at least a distal housing face 142 of lower portion 132 is preferably transparent so as to allow the user to observe the target skin site during the testing process. Various views of distal face 142 are illustrated in Figs. 7A-7C and 8. Within pressure ring 144 is a slot 180 through which the microneedle 172 of a dispensed tester extends when in a testing or active position, as illustrated in Figs. 7C and 8. As best shown in Fig. 7B, distal housing face further includes a set of electrical contacts or leads 184a and 184b which extend from the meter's electronics which are housed in upper portion 130. Such electronics typically include a controller in the form of a microprocessor for controlling all electronic related system functions, including but not limited to, displaying information, receiving data input by the user, implementing user commands, the testing of the physiological fluid (*e.g.*, sending the necessary electrical signals to the tester), receiving calibration information from the cassette, storing system software and information into memory, etc. Electrical contacts 184 are aligned to engage with the corresponding electrodes or electrode contacts of the testers. As such, input signals are provided to and output signals are received from the tester's sensor for testing, for example, the analyte concentration of the sampled fluid. Further electrical contacts are spring-loaded against a tester guiding member 188 such that when a tester 172 is dispensed in a testing position, as shown in Fig. 7C, it is securely held so as to remain in position and resist any flexing which may occur as a result of penetrating the skin..

### Methods of the Present Invention

The methods of the present invention involve the use of meter cassette 102 and meter 104. To operate meter 104, the user begins by depressing button 140, as illustrated in Figs. 4B, 4C and 4D, to unlock a locking mechanism (not shown) which maintains upper portion 130 and lower portion 132 in a locked position when meter 104 is not in use to prevent inadvertent activation of the meter. Depression of button 140 could also serve to turn on the meter's electronics.

In operation, lower portion 132 is slidingly received by upper portion 130. Thus, when distal face 142 of lower body portion 132 is pressed against the skin, lower portion 132 moves in the direction of arrow 145 of Fig. 4D while being resisted by a spring (not shown) within the meter. The load of the spring may be adjustable to accommodate various test locations on the body and the needs of different users. When lower portion 132 has traveled a predetermined distance relative to upper portion 130, another spring mechanism, which has become loaded or energized by this relative motion, is unlatched thereby forcing cassette 102 to move downward within meter 104. During the cassette's travel, gear drive 152 of cassette 102 (see Fig. 4A) engages with geared tooth rack 156 causing gear drive 152 to rotate in the direction of arrow 154. This in turn causes drive roller 118 to rotate in the direction of arrow 122a (see Fig. 5) which, in turn, rotates drive roller 120 in the direction of arrow 122b. The counter rotation of drive rollers 118 and 120 drives strip 106, by means of the engagement of sprockets 164 within sprocket holes 166, in the direction of 128a, causing the next unused tester 130a to enter through throat 116 between rollers 118 and 120. Top and bottom portions 126 and 134 of the packaging which encase tester 130a are pulled apart and fed into there respective tracks 110 and 113. Tester 130a is then optimally oriented to contact electrode contacts 184a and 184b of distal housing face 142 of meter 104, as illustrated in Fig. 7C, and to penetrate the skin with microneedle 172. If for some reason, lower portion 132 does not travel the predetermined distance, *i.e.,* the user fails to complete the necessary actuation of meter 104, the packaging encasing unused tester 130a will remain sealed so as not to expose it to contaminants. Concurrently with the dispensing of unused tester 130a, the last used tester 130b is caused to rotate with drive roller 118 and fold back into its corresponding recess of strip 106 and fed along with it into track 113.

The penetration depth of microneedle 172 is preferably set to between about 0.02 mm and 2.0 mm, or more preferably set between 0.5 mm and 1.5 mm. In addition to placing pressure about the needle penetration site, pressure ring 144 results at least in part from stretching the skin in this area and thereby helps to extract a sample from the skin and "pump" it into the fluid transfer channel within microneedle 172. The sampled fluid is then transferred to the sensor portion of tester 130awherein an electrochemical assay is made, and the results displayed on display screen 136 of meter 104.

The user continues to hold meter 104 in stable position until the meter signals the user to remove the needle from the skin. Such a signal is given once an adequate sample volume of physiological fluid has been received within the tester's sensor. For example, meter 104 may be configured to display a "sample received" icon to indicate to the user that a sufficient volume of fluid has been collected and the meter may now be removed from the test site.

Removing the distal housing surface 142 from the skin releases lower housing portion 132 to return to an extended, locked position and disengages geared tooth rack 156 from drive gear 152, thereby preventing drive gear 152 from rotating strip drive rollers 118 and 120. In this extended, locked position, distal housing face 142 extends beyond the tip of microneedle 172, thus, preventing inadvertent contact with it by the user. Protective cap 134 may then be positioned over lower housing portion 132 while system 100 is not in use. An example of a meter having an upper and lower housing interface similar to that of meter 104 is described in European patent application No. , claiming priority from USSN 10/142,443, filed on 9th May 2002 [Attorney ref: P033752EP].

The above steps are repeated as necessary when desiring to measure a target analyte. Upon the subsequent indexing of strip 106, tester 130a is caused to advance around roller 118 and returned to a seated position within its corresponding recess, while the next unused tester is operatively dispensed. As the testers are successively used, the used portion of strip 106 advances through tracks 110 and 113, and is caused to be spooled within cassette 102 until all testers are used. The completely used cassette 102 is removed from meter 104 and may be disposed of without concern of creating a biohazard. A cassette containing a partially used strip of testers may be removed from the meter and later engaged to use the remaining unused testers.

### Kits

Also provided by the present invention are kits for practicing the subject methods. In one embodiment, the kits include a subject system for practicing the subject invention. The subject system includes a subject meter and at least one subject cassette which may be disposable and replaceable. The subject cassette may be pre-loaded with a strip of testers as described above or may be provided. Additionally, the kit may include one or more strips to be loaded into the cassette. Finally, the kits typically include instructions for using the subject systems and for loading and removing cassettes into and out of the subject meters. These instructions may be present on one or more of the packaging, a label insert, containers present in the kits, and the like.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

## Claims

1. A packaging for containing testers for accessing, collecting and analyzing a sample of fluid, comprising:
a first strip portion having a length dimension and a width dimension and comprising a plurality of serially aligned recesses, each said recess comprising an opening for receiving a tester therein and configured for containing said tester therein;
a second strip portion having length and width dimensions similar to said length and said width dimensions of said first strip portion, wherein said second strip portion covers said opening of each said recess; and **characterized in that** it comprises
means for affixing a tester relative to each of said corresponding recesses.

2. A method of packaging testers for accessing, collecting and analyzing a sample of fluid, comprising the steps of:
providing a plurality of testers to be packaged;
providing the packaging of claim 1;
associating each of said plurality of testers at the opening of a recess of said first strip portion;
attaching said plurality of testers to said first strip portion; and
sealing said second strip portion to said first strip portion wherein said testers are sealed within said associated recess.

3. The packaging of claim 1, wherein the second strip portion is capable of hermetically sealing each said tester within said corresponding recess.

4. The packaging of claim 1, further comprising:
a cassette for operatively containing and dispensing testers, said cassette comprising:
a housing;
at least one track defined within said housing for storing and guiding a strip of testers when loaded within said cassette;
a pair of drive rollers for indexing said strip of testers; and
a tester dispensing space defined between said pair of drive rollers wherein only a single tester is dispensable through said tester dispensing port at a time.

5. A system for accessing and collecting a sample of physiological fluid and for measuring the concentration of a target analyte within said fluid, said system comprising:
the packaging according to claim 4; and
a meter comprising:
(i) a housing having a receptacle for receiving said cassette therein and having a distal face having a slot aligned with said tester dispensing space; and
(ii) means for activating said drive rollers of said cassette.

6. A method of dispensing testers from a strip of testers for accessing and collecting fluid from a target skin surface, comprising the steps of:
(a) providing the packaging of claim 1, wherein each of said recesses has a tester affixed thereto; and
(b) separating the first strip portion from the second strip portion, wherein only a single tester is exposed from an associated recess.

7. The packaging of claim 1, wherein the means for retaining a tester comprises:
a frame structure having a length, wherein a plurality of testers may be flexibly attached to said frame serially aligned along said length.

8. A method of dispensing testers comprising the steps of:
(a) providing said packaging of claim 4;
(b) operatively loading said strip within said at least one track;
(c) indexing said strip towards said tester dispensing space;
(d) separating said first strip portion from said second strip portion of said strip;
(e) dispensing a first tester through said tester dispensing space.

9. A kit for accessing, collecting and analyzing a sample of fluid, comprising:
the packaging of claim 1; and
at least one cassette for dispensing the testers from the packaging.

## Patentansprüche

1. Verpackung zum Halten von Testern zum Zugreifen, Sammeln und Analysieren einer Probe eines Fluids, mit:
einem ersten Streifenabschnitt mit einer Längsdimension und einer Breitendimension und mit mehreren seriell ausgerichteten Vertiefungen, von denen jede eine Öffnung zum Aufnehmen eines Testers umfaßt und konfiguriert ist, den Tester hierin zu halten;
einem zweiten Streifenabschnitt mit Längen- und Breitendimensionen ähnlich den Längen- und Breitendimensionen des ersten Streifenabschnitts, wobei der zweite Streifenabschnitt die Öffnung jeder Vertiefung bedeckt; **gekennzeichnet durch** Mittel zum Anbringen eines Testers relativ zu jeder der zugehörigen Vertiefungen.

2. Verfahren zum Verpacken von Testern zum Zugreifen, Sammeln und Analysieren einer Probe eines Fluids, wobei das Verfahren die folgenden Schritte umfaßt:
Bereitstellen mehrerer zu verpackender Tester;
Vorsehen einer Verpackung nach Anspruch 1;
Zuordnen jedes der mehreren Tester zu der Öffnung einer Vertiefung des ersten Streifensabschnitts;
Anbringen der mehreren Tester an dem ersten Streifenabschnitt; und
Verbinden des zweiten Streifenabschnitts mit dem ersten Streifenabschnitt, wobei die Tester in der zugehörigen Vertiefung eingeschlossen werden.

3. Verpackung nach Anspruch 1, **dadurch gekennzeichnet, daß** der zweite Streifenabschnitt in der Lage ist, jeden Tester in der zugehörigen Vertiefung hermetisch einzuschließen.

4. Verpackung nach Anspruch 1, **gekennzeichnet durch**:
eine Kassette, welche ausgebildet ist, Tester zu halten und abzugeben, die Kassette umfassend:
ein Gehäuse;
zumindest eine in dem Gehäuse gebildete Bahn zum Lagern und Führen eines Streifens von Testern, wenn diese in diese Kassette geladen sind;
ein Paar von Antriebsrollen zum Führen des Streifens von Testern; und
einen zwischen dem Paar von Antriebsrollen gebildeten Testerabgaberaum, wobei nur ein einzelner Tester **durch** die Testerabgabeöffnung abgegeben werden kann.

5. System zum Zugreifen und Sammeln einer Probe eines physiologischen Fluids und zum Messen der Konzentration eines Zielanalyts in dem Fluid, mit:
der Verpackung nach Anspruch 4; und
einem Meßelement mit:
(i) einem Gehäuse mit einer Aufnahme zum Aufnehmen der Kassette hierin und einer distalen Fläche mit einem mit dem Testerabgaberaum ausgerichteten Schlitz; und
(ii) Mittel zum Aktivieren der Antriebsrollen der Kassette.

6. Verfahren zum Abgeben von Testern von einem Streifen von Testern zum Zugreifen und Sammeln eines Fluids aus einer Ziel-Hautoberfläche, wobei das Verfahren die folgenden Schritte umfaßt:
(a) Bereitstellen einer Verpackung nach Anspruch 1, wobei an jede der Vertiefungen ein Tester angebracht ist; und
(b) Trennen des ersten Steifensabschnitts vom zweiten Streifenabschnitt, wobei nur ein einzelner Tester aus einer zugehörigen Vertiefung freigelegt wird.

7. Verpackung nach Anspruch 1, **dadurch gekennzeichnet, daß** das Mittel zum Halten eines Testers eine Rahmenstruktur mit einer Länge umfaßt, wobei mehrere Tester flexibel an dem Rahmen befestigt, seriell entlang der Länge ausgerichtet sein können.

8. Verfahren zum Abgeben von Testern, wobei das Verfahren die folgenden Schritte umfaßt:
(a) Bereitstellen der Verpackung nach Anspruch 4;
(b) Laden des Streifens in die zumindest eine Bahn;
(c) Führen des Streifens in Richtung des Testerabgaberaumes;
(d) Trennen des ersten Streifensabschnitts vom zweiten Streifenabschnitt des Streifens; und
(e) Abgeben eines ersten Testers durch den Testerabgaberaum.

9. Anordnung zum Zugreifen, Sammeln und Analysieren einer Probe eines Fluids, mit:
der Verpackung nach Anspruch 1; und
mindestens einer Kassette zum Abgeben der Tester aus der Verpackung.

## Revendications

1. Emballage contenant des testeurs pour accéder, recueillir et analyser un échantillon de fluide, comprenant
une première portion de bande ayant une dimension en longueur et une dimension en largeur et comprenant plusieurs évidements alignés en série, chacun desdits évidements comprenant une ouverture pour recevoir un testeur dans celle-ci et configurée pour contenir ledit testeur dans celle-ci ;
une deuxième portion de bande ayant des dimensions de longueur et de largeur similaires auxdits dimensions de longueur et de largeur de ladite première portion de bande, où ladite deuxième portion de bande couvre ladite ouverture de chacun desdits évidements ; et **caractérisé en ce qu'**il comprend
un moyen pour fixer un testeur relativement à chacun desdits évidements correspondants.

2. Procédé d'emballage de testeurs pour accéder, recueillir et analyser un échantillon de fluide, comprenant les étapes consistant à :
réaliser une pluralité de testeurs à emballer ;
réaliser l'emballage de la revendication 1 ;
associer chacun de ladite pluralité de testeurs à l'ouverture d'un évidement de ladite première portion de bande ;
fixer ladite pluralité de testeurs à ladite première portion de bande ; et
sceller ladite seconde portion de bande à ladite première portion de bande où lesdits testeurs sont scellés dans lesdits évidements associés.

3. Emballage selon la revendication 1, où ladite seconde portion de bande est apte à sceller hermétiquement chaque testeur précité dans ledit évidement correspondant.

4. Emballage selon la revendication 1, comprenant en outre :
une cassette pour contenir et distribuer fonctionnellement des testeurs, ladite cassette comprenant :
un boîtier ;
au moins un chemin défini dans ledit boîtier pour stocker et guider une bande de testeurs lorsqu'elle est chargée dans ladite cassette ;
une paire de rouleaux d'entraînement pour indexer ladite bande de testeurs ; et
un espace de distribution de testeurs défini entre ladite paire de rouleaux d'entraînement où seulement un seul testeur peut être distribué à travers ledit orifice de distribution de testeurs à la fois.

5. Système pour accéder et recueillir un échantillon de fluide physiologique et pour mesurer la concentration d'une substance à analyser cible dans ledit fluide, ledit système comprenant :
l'emballage selon la revendication 4 ; et
un mètre comprenant :
(i) un boîtier avec un récipient pour recevoir ladite cassette et ayant une face distale avec une fente alignée avec ledit espace de distribution de testeurs ; et
(ii) un moyen pour activer lesdits rouleaux d'entraînement de ladite cassette.

6. Procédé pour distribuer des testeurs d'une bande de testeurs pour accéder et recueillir un fluide d'une surface de peau cible, comprenant les étapes consistant à :
(a) réaliser l'emballage selon la revendication 1, où chacun desdits évidements à un testeur fixé à celui-ci ; et
(b) séparer la première portion de bande de la seconde portion de bande, où un seul testeur est exposé d'un évidement associé.

7. Emballage selon la revendication 1, où le moyen pour retenir un testeur comprend :
une structure de châssis ayant une longueur, où une pluralité de testeurs peut être fixée d'une manière flexible audit châssis alignée en série sur ladite longueur.

8. Procédé de distribution de testeurs comprenant les étapes consistant à :
(a) réaliser ledit emballage selon la figure 4 ;
(b) charger fonctionnellement ladite bande dans au moins un chemin ;
(c) indexer ladite bande vers ledit espace de distribution de testeurs ;
(d) séparer ladite première portion de bande de ladite seconde portion de bande de ladite bande ;
(e) distribuer un premier testeur à travers ledit espace de distribution de testeurs.

9. Kit pour accéder, recueillir et analyser un échantillon de fluide, comprenant :
l'emballage de la revendication 1 ; et
au moins une cassette pour distribuer les testeurs de l'emballage.
